(19) Europäisches Patentamt
European Patent Office
Office européen des brevets

(11) Veröffentlichungsnummer: **0 000 763**
**A1**

(12) # EUROPÄISCHE PATENTANMELDUNG

(21) Anmeldenummer: 78100563.2

(22) Anmeldetag: 01.08.78

(51) Int. Cl.$^2$: C 07 D 303/36

(30) Priorität: 09.08.77 DE 2735765

(43) Veröffentlichungstag der Anmeldung: 21.02.79 Patentblatt 79/4

(84) Benannte Vertragsstaaten: BE CH DE FR GB NL SE

(71) Anmelder: BASF Aktiengesellschaft
Carl-Bosch-Strasse 38
D-6700 Ludwigshafen(DE)

(72) Erfinder: Strickler, Rainer, Dr.
Schroederstrasse 14
D-6900 Heidelberg(DE)

(72) Erfinder: Oppenlaender, Knut, Dr.
Otto-Dill-Strasse 23
D-6700 Ludwigshafen(DE)

(72) Erfinder: Merkel, Karl, Dr.
Sperlinggasse 15
D-6700 Ludwigshafen(DE)

(72) Erfinder: Penzel, Erich, Dr.
Carl-Bosch-Strasse 86
D-6700 Ludwigshafen(DE)

(72) Erfinder: Fischer, Robert, Dr.
Curt-Schumann-Ring 18
D-6701 Maxdorf 2(DE)

(54) **Glycidylätheramine und Verfahren zu ihrer Herstellung.**

(57) Diese Erfindung betrifft ein Verfahren zur Herstellung von Clycidylätheraminen der allgemeinen Formel (I)

$$X_2N-R^1-(O-R^2-)_nO-R^1-NX_2$$

in der

X für H und/oder $-CH_2-\underbrace{CH-CH_2}_{O}$ mit mindestens 2 $-CH_2-\underbrace{CH-CH_2}_{O}$ je Mol,

$R^1$ für 1 bis 4 C-Atome enthaltende Alkylengruppen,

$R^2$ für 1 bis 20 C-Atome enthaltende Alkylengruppen und

n für 0 bis 50

stehen durch Umsetzen von Ätherdiaminen der allgemeinen Formel (II)

$$H_2N-R^1-(O-R^2-)_n-O-R^1-NH_2,$$

in der $R^1$, $R^2$ und n die oben angegebenen Bedeutungen haben, mit Epichlorhydrin bei erhöhter Temperatur, wobei zunächst (II) mit überschüssigem Epichlorhydrin bei Temperaturen von 25 bis 40°C in Gegenwart von mindestens 0,5 Gew.% Wasser umgesetzt und das Reaktionsgemisch dann mit Alkali bei Temperaturen unter 50°C versetzt wird.

Die Erfindung betrifft zudem Glydicylätheramine der allgemeinen Formel (I), in der X ausschließlich für $-CH_2-\underbrace{CH-CH_2}_{O}$, $R^1$ für 2 bis 3 C-Atome enthaltende Alkylengruppen, $R^2$ für 2 bis 6 C-Atome enthaltende Alkylengruppen und n für 0 bis 15 stehen, die besonders wirksame Vernetzer für coreaktive Gruppen aufweisende Polymerisate sind.

EP 0 000 763 A1

Croydon Printing Company Ltd.

## Glycidylätheramine und Verfahren zu ihrer Herstellung

Aus der DE-PS 11 32 146, der US-PS 3 278 561 und J. Org. Chem. 28, 2283 (1963) ist es schon bekannt, daß man primäre und sekundäre Diamine mit Epichlorhydrin umsetzen kann, wobei man zunächst die 3-Chlor-2-oxypropylamine und daraus unter der Einwirkung wäßriger Alkalilaugen Gylcidylamine erhält. Nach dem Verfahren der US-PS 2 963 482 können durch Umsetzung von sekundären Diaminen, wie Piperazin, durch Umsetzung mit Epichlorhydrin und Alkalilaugen N,N'-Bis-2,3-epoxypropyldiamine erhalten werden.

Außerdem ist aus der britischen Patentschrift 745 499 bekannt, daß man Polyamine mit Epichlorhydrin oder Dichlorhydrin in Gegenwart von Alkali bei erhöhter Temperatur umsetzen kann. Dabei kann auch $\gamma, \gamma'$-Diaminopropyläther anstelle von Diaminen bei einer Reaktionstemperatur von 50°C in Abwesenheit von Alkali eingesetzt werden, wobei man Polyadditionsprodukte erhält, die zur Naßverfestigung von Papier eingesetzt werden können. Schließlich ist es aus der US-PS 3 312 664 bekannt, Diaminomethyl-diphenyloxide mit Epichlorhydrin bei etwa 70°C umzusetzen und dann Alkalilauge zuzusetzen, wobei N,N'-Tetraäthoxypropyldiaminomethyl-diphenyloxide erhalten werden. Aus diesen

Wd/Fe

Tetraglycidylverbindungen können unter Verwendung der üblichen Härter Epoxyharze mit verhältnismäßig guter Temperaturbeständigkeit hergestellt werden.

Es wurde nun gefunden, daß man Gylcidylätheramine der allgemeinen Formel (I)

$$X_2N-R^1-(O-R^2-)_nO-R^1-NX_2 \quad ,$$

in der X für H und/oder $-CH_2-\underbrace{CH-CH_2}_{O}$ mit mindestens zwei $-CH_2-\underbrace{CH-CH_2}_{O}$ je Molekül, $R^1$ für 1 bis 4 C-Atome enthaltende Alkylengruppen, $R^2$ für 1 bis 20 C-Atome enthaltende Alkylengruppen und n für 0 bis 50 stehen, durch Umsetzen von Ätherdiaminen der allgemeinen Formel (II)

$$H_2N-R^1-(O-R^2-)_n-O-R^1-NH_2 \quad ,$$

in der $R^1$, $R^2$ und n die oben angegebenen Bedeutungen haben, mit Epichlorhydrin bei erhöhter Temperatur mit Vorteil umsetzen kann, wenn man zunächst (II) mit 2,4 bis 7 Mol Epichlorhydrin je Mol (II) bei Temperaturen von 25 bis 40°C in Gegenwart von mindestens 0,5 Gew.%, bezogen auf die Menge an (II) Wasser umsetzt und das Reaktionsgemisch dann mit der zur Menge des eingesetzten Epichlorhydrins mindestens gleichen molaren Menge Alkali bei Temperaturen unter 50°C versetzt. Je nach der eingesetzten Menge des Epichlorhydrins werden 2, 3 oder alle Amin-Wasserstoffatome der Ätherdiamine (II) durch 2,3-Epoxypropyl-Gruppen substituiert, und die Glycidylverbindungen fallen im allgemeinen als Gemische an. Von den Glycidylätheraminen der allgemeinen Formel (I) sind die Tetraglycidylätheramine von besonderem Interesse. In ihnen bedeutet X ausschließlich den 2,3-Epoxypropylrest ($-CH_2-\underbrace{CH-CH_2}_{O}$) und

$R^1$ steht vorzugsweise für 2 bis 3 C-Atome enthaltende Alkylengruppen, insbesondere für Trimethylengruppen, $R^2$ vorzugsweise für 2 bis 6 C-Atome enthaltende Alkylengruppen, insbesondere für den Äthyliden-, Trimethylen-, Tetramethylen-, Pentamethylen- und/oder Hexamethylenrest, und n für avorzugsweise 0 bis 15. Geeignete Ätherdiamine (II) zur Herstellung der Glycidylätheramine (I) sind beispielsweise 4,7,10-Trioxatridecan-1,13-diamin, 4,7-Dioxadecan-1,10-diamin, 4,9-Dioxadodecan-1,12-diamin, 4-Oxaheptan-1,7-diamin und 4,11-Dioxatetradecan-1,14-diamin, 4-Oxaheptan-1,7-diamin, 4,11-Dioxatetradecan-1,14-diamin und das Ätheramin der allgemeinen Formel $H_2NC_3H_6C(C_4H_8O)_9,C_3H_6NH_2$.

Bei der Umsetzung der Ätheramine (II) mit Epichlorhydrin arbeitet man im allgemeinen bei Temperaturen von 25 bis 40, vorzugsweise bei 30 bis 35°C in Gegenwart von Spuren Wasser. Im allgemeinen sollen mindestens etwa 0,5, insbesondere 1 % Wasser, bezogen auf die Menge des theramins (II) zugegen sein und die Temperatur soll 50°C nicht erreichen, um die Bildung unerwünschter Polykondensationsprodukte zu verhindern. Es hat sich als vorteilhaft erwiesen, die Umsetzung des Epichlorhydrins in einem Lösungsmittel, vorzugsweise in meist 1 bis 3 C-Atome aufweisenden Alkanolen wie Methanol, Äthanol, Isopropylalkohol oder n-Propylalkohol oder auch in Wasser oder Gemischen aus Wasser und Alkanolen der genannten Art durchzuführen, wobei die Konzentration des Epichlorhydrins in dem Lösungsmittel in weiten Grenzen variiert werden kann. Meist liegt sie zwischen 0,1 und 25 %, vorzugsweise zwischen 0,5 und 5 Gew.%, bezogen auf die Lösung. Zu der Lösung des Epichlorhydrins wird bei dem bevorzugten Verfahren das Ätherdiamin (II), gegebenenfalls in Form seiner Lösung, in einem Lösungsmittel der genannten Art, d.h. einem

Alkanol und/oder Wasser zufließen lassen, wobei meist gekühlt wird. Die Zugabe der vorzugsweise wäßrigen Alkalilauge, deren Konzentration an Alkali, bezogen auf die Lauge, meist zwischen 10 und 50, vorzugsweise zwischen 30 und 45 Gew.% liegt, erfolgt vorzugsweise, wenn das Epichlorhydrin praktisch vollständig mit dem Ätheramin ausreagiert hat. Dabei wird die Reaktionstemperatur, gegebenenfalls durch Kühlung, unter 50°C, vorzugsweise zwischen 0° und 40°, insbesondere zwischen 20 und 30°C gehalten. Bei der Umsetzung der Ätheramine (II) mit Epichlorhydrin entstehen im Reaktionsgemisch dei entsprechenden N-Chlorhydrine, die im allgemeinen nicht isoliert werden, und aus denen dann unter der Einwirkung der Alkalilauge unter Abspaltung von HCl die entsprechenden Glycidylverbindungen entstehen.

Aus dem Reaktionsgemisch können die Glycidylätheramine beispielsweise unter Verwendung vonflüssigen Kohlenwasserstoffen, wie Benzol, Pentan oder Toluol oder von Äthern, wie Diäthyläther und Diisobutyläther extrahiert werden. Aus dem Extrakt können die Glycidylätheramine nach dem Trocknen beispielsweise durch Abdampfen des Extraktionsmittels (Äther oder Kohlenwasserstoff) als Rückstand gewonnen werden. Ihre Ausbeute, bezogen auf das eingesetzte Ätherdiamin (II), liegt im allgemeinen zwischen 50 und 80 % der Theorie und die Produkte können bei Temperaturen bis 20°C, insbesondere unter Zusatz üblicher Stabilisierungsmittel, praktisch unzersetzt mehrere Wochen gelagert werden. Für die Lagerung ist es von Vorteil, die Glycidylätheramine als Lösung in Dioxan oder Äthylenglykoldimethyläther aufzubewahren.

Nach dem erfindungsgemäßen Verfahren wurden z.B. folgende Tetraglycidylätheramine hergestellt:

Tetraglycidyl-4,7-dioxadecan-1,10-diamin
(Poxid-O: 15,5 %, Theorie 15,8 %)

Tetraglycidyl-4,9-dioxadodecan-1,12-diamin
(Epoxid-O: 14,6 %, Theorie: 14,8 %).

Alle weiteren Tetraglycidylamine (siehe Tabelle) wurden nur als Rohprodukte mit ca. 70 % Gehalt an Hauptkomponente erhalten und nicht in die Einzelkomponenten aufgetrennt.

Die Glycidylätheramine sind im allgemeinen niedrigviskose farblose bis hellgelbe Öle. Sie sind in vielen organischen Lösungsmitteln, z.B. in Benzol, Toluol, Tetrahydrofuran, Dioxan, Methylalkohol, Äthylalkohol, Cyclohexanon und Dimethylformamid leicht löslich. Außerdem sind die Glycidylätheramine meist wasserlöslich, insbesondere soweit ihre Kohlenstoffzahl im Rest $R^2$ nicht über 2 liegt. Sie reagieren mit Amino-, Alkohol-, Carbonsäure-, Anhydrid- und Phenolgruppen. Sie eignen sich beispielsweise als Vernetzer für coreaktive Gruppen aufweisende Polymerisate und übertreffen dabei die sich von primären aliphatischen Aminen ableitenden Diglycidylamine, wie n-Butyl-bis-glycidylamin. Außerdem können sie als Mittel für die Regulierung der Konsistenz von Siebdruckfarben, insbesondere auf Basis von Äthylenglykol, eingesetzt werden.

Die neuen aliphatischen Tetraglycidylätheramine sind im Vergleich zu den araliphatischen Tetraglycidylätheraminen der US-PS 3 312 664 wesentlich reaktionsfähiger und wesentlich weniger lichtempfindlich. Außerdem übertreffen sie die Tetraglycidylverbindungen der US-PS 3 312 664 in ihrer Wasserlöslichkeit, was bei vielen Reaktionen von Vorteil ist.

Die in den folgenden Beispielen angegebenen Teile sind Gewichtsteile. Die darin angegebenen Volumenteile verhalten sich dazu wie Liter zu Kilogramm.

Beispiel 1

In einem Reaktionsgefäß, das mit Thermometer, Zulaufgefäß und Rührer ausgestattet ist, legt man ein Gemisch von 378 Teilen Epichlorhydrin und 50 Volumenteilen Wasser vor. Man kühlt von außen mit Eiswasser und dosiert 240 Teile 4,7,10-Trioxatridecan-1,13-diamin (Reinheitsgrad 91 %) dararart zu, daß die Temperatur des Reaktionsgemisches zwischen 30 und 35°C bleibt. Bei dieser Temperatur rührt man nach Zugabe des Diamins noch 4 Stunden nach, bis der Gehalt an Epoxid-Sauerstoff der Reaktionslösung unter 0,4 % gesunken ist.. Man verdünnt dann mit 200 Volumenteilen Isopropanol und gibt insgesamt 400 Volumenteil 50 %ige wäßrige Natronlauge im Verlauf einer halben Stunde bei 20 bis 25°C zu. Man rührt anschließend noch 2 Stunden, saugt von ausgefallenem Natriumchlorid ab und extrahiert zweimal mit je 500 Volumenteilen Toluol. Die vereinigten Toluol-Extrakte werden über Kaliumhydroxid, dann über Magnesiumsulfat getrocknet. Von den getrockneten Extrakten wird das Toluol unter vermindertem Druck bei 60°C abgedampft. Als Rückstand erhält man 322 Teile eines Gemisches aus N-Glycidyl-Verbindungen des 4,7,10-Trioxatridecan-1,13-diamins, das, wie sich aus den Angaben über den Poxidgehalt in der folgenden Tabelle ergibt, überwiegend aus Tetraglycidyl-4,7,10-trioxatridecan-1,13-diamin besteht.

Beispiele 2 bis 6

Man arbeitet wie in Beispiel 1 angegeben, wobei man gleichfalls jeweils je Mol der in der Tabelle angegebenen Ätherdiamine 4,1 Mol Epichlorhydrin einsetzt. Die Ergebnisse

sind gleichfalls in der folgenden Tabelle 1 angegeben.

Tabelle 1

| Bsp. Nr. | eingesetztes Amin | Polyepoxypropylamin $X = CH_2\underset{O}{CH-CH_2}$ bzw. H | Ausbeute an reinem Tetraepoxy-G propylamin % | Epoxid O % | theor. Epoxid O % für alle $X = CH_2\underset{O}{CHCH_2}$ | Reinheit % für alle $X = CH_2\underset{O}{CHCH_2}$ | Ausbeute % für alle $X = CH_2\underset{O}{CHCH_2}$ |
|---|---|---|---|---|---|---|---|
| 1 | 4,7,10-Trioxatridecan-1,13-diamin (91 proz.) $H_2NC_3H_6O(CH_2CH_2O)_2C_3H_6NH_2$ | $X_2NC_3H_6O(CH_2CH_2O)_2C_3H_6NX_2$ | | 12,8 | 14,4 | 89 | 64 |
| 2 | 4,7-Dioxadecan-1,10-diamin (96 proz.) $H_2NC_3H_6O(CH_2CH_2O)C_3H_6NH_2$ | $X_2NC_3H_6OCH_2CH_2OC_3H_6NX_2$ | 48 | 13,7 | 15,9 | 89 | 62 |
| 3 | 4,9-Dioxadodecan-1,12-diamin (95 proz.) $H_2NC_3H_6OC_4H_8OC_3H_6NH_2$ | $X_2NC_3H_6OC_4H_8OC_3H_6NX_2$ | 60 | 11,5 | 14,8 | 78 | 67 |
| 4 | 4-Oxaheptan-1,7-diamin $H_2NC_3H_6OC_3H_6NH_2$ | $X_2NC_3H_6OC_3H_6NX_2$ | | 14,1 | 18,0 | 79 | 72 |
| 5 | 4,11-Dioxatetradecan-1,14-diamin $H_2NC_3H_6OC_6H_{12}OC_3H_6NH_2$ | $X_2NC_3H_6OC_6H_{12}OC_3H_6NX_2$ | | 10,2 | 13,9 | 74 | 57 |
| 6 | Diätheramin aus Poly-THF 650 $H_2NC_3H_6O(C_4H_8O)_9C_3H_6NH_2$ | $X_2NC_3H_6O(C_4H_8O)_9C_3H_6NX_2$ | | 4,3 | 6,3 | 68 | 57 |

Aus den Reaktionsprodukten der Beispiel 2 und 3 wurden über Flüssigkeitschromatographie (Gelpermeation) auf einer Merkogel OR-PVA-2000-Säule mit Tetrahydrofuran als Elutionsmittel und einem Differentialrefraktometer als Detektor die entsprechenden Tetraglycidylätheramine isoliert. Im Reaktionsgemisch des Beispiels 2 wurden 68 % Tetraglycidyl-4,6-dioxadecan-1,10-diamin vom Epoxid-O-Gehalt 15,5 % (Theorie 15,8) und Stickstoffgehalt 6,7 % (Theorie 6,9 %) gefunden; 30 % des Produkts bestanden aus höhermolekularen Komponenten, die ebenfalls Epoxid enthielten. Zur Epoxid- und Stickstoffbestimmung wurde die Hauptkomponente über präparative Flüssigkeitschromatographie isoliert.

Nach dem agleichen Verfahren wurden aus dem Produkt von Beispiel 3

71 % Tetraglycidyl-4,9-dioxadodecan-1,12-diamin
mit 14,6 % Epoxid-O (Theorie 14,8 %)
und 6,3 % Stickstoff (Theorie 6,5 %)

isoliert.

## Patentansprüche

1. Verfahren zur Herstellung von Glycidylätheraminen der allgemeinen Formel (I)

$$X_2N-R^1-(O-R^2-)_nO-R^1-NX_2 \quad ,$$

in der

X für H und/oder 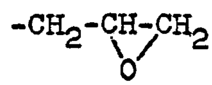 mit mindestens

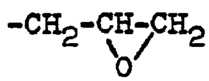 je Mol,

$R^1$ für 1 bis 4 C-Atome enthaltende Alkylengruppen,

$R^2$ für 1 bis 20 C-Atome enthaltende Alkylengruppen und

n für 0 bis 50

stehen, durch Umsetzen von Ätherdiaminen der allgemeinen Formel (II)

$$H_2N-R^2-(O-R^2-)_n-O-R^1-NH_2 \quad ,$$

in der $R^1$, $R^2$ und n die oben angegebenen Bedeutungen haben, mit Epichlorhydrin bei erhöhter Temperatur, dadurch gekennzeichnet, daß zunächst (II) mit 2,4 bis 7,0 Mol Epichlorhydrin je Mol (II) bei Temperaturen von 25 bis 40°C in Gegenwart von mindestens 0,5 Gew.%, bezogen auf die Menge an (II), Wasser umgesetzt und das Reaktionsgemisch dann mit der zur Menge des eingesetzten Epichlorhydrins mindestens gleichen molaren Menge Alkali bei Temperaturen unter 50°C versetzt wird.

2. Glycidylätheramine der allgemeinen Formel (I), in der

X ausschließlich für 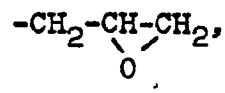,

$R^1$ für 2 bis 3 C-Atome enthaltende Alkylengruppen,

$R^2$ für 2 bis 6 C-Atome enthaltende Alkylengruppen und

n für 0 bis 15

stehen.

0000763

Europäisches Patentamt

# EUROPÄISCHER RECHERCHENBERICHT

Nummer der Anmeldung

EP 78 10 0563

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | CH - A - 396 034 (UNION CARBIDE)<br>* Seite 2, rechte Spalte, Zeilen 37-45; Seiten 5,6; Patentansprüche *<br>-- | 1 | C 07 D 303/36 |
| D | GB - A - 745 499 (BAYER)<br>* Gesamte Patentschrift *<br>---- | 1,2 | |
| | | | RECHERCHIERTE SACHGEBIETE (Int. Cl.²) |
| | | | C 07 D 303/36 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 14-11-1978 | ALLARD |